# EUROPEAN PATENT APPLICATION

(11) **EP 4 803 011 A1**
(43) Date of publication of application: **09.09.2026**
(21) Application number: 26162525.5
(22) Date of filing: 05.03.2026
(51) Int. Cl.: A61B 8/06, A61B 8/08, A61B 8/00

(54) **ULTRASOUND DIAGNOSTIC APPARATUS AND CONTROL METHOD OF ULTRASOUND DIAGNOSTIC APPARATUS**

(30) Priority: 06.03.2025 JP 2025035579
(71) Applicant: FUJIFILM Corporation, Tokyo 106-8620 (JP)
(72) Inventor: MATSUMOTO, Tsuyoshi, Kaisei (JP)
(74) Representative: Parker, Andrew James

(57) **Abstract**

Provided are an ultrasound diagnostic apparatus and a control method of an ultrasound diagnostic apparatus that can easily and accurately select an ultrasound image of a frame suitable for measuring a diameter of a left ventricular outflow tract.

The ultrasound diagnostic apparatus that guides a frame suitable for measuring a diameter of a left ventricular outflow tract from ultrasound images of a plurality of frames in which a heart of a subject is imaged includes an image recognition unit that performs image recognition on a local anatomical structure of the heart in each of the plurality of frames, a time-varying waveform generation unit that generates a time-varying waveform of an anatomical structure state based on the local anatomical structure recognized by the image recognition unit, a candidate frame extraction unit that extracts a measurement candidate frame from the plurality of frames using the time-varying waveform, a monitor, and a display controller that displays the measurement candidate frame in a highlighted manner on the monitor.

## Description

### BACKGROUND OF THE INVENTION

### 1. Field of the Invention

The present invention relates to an ultrasound diagnostic apparatus that images a heart of a subject and a control method of the ultrasound diagnostic apparatus.

### 2. Description of the Related Art

In the related art, a so-called cardiac output is calculated by capturing an ultrasound image representing a tomographic plane of a heart of a subject using a so-called ultrasound diagnostic apparatus and analyzing the captured ultrasound image. The cardiac output is usually calculated by performing calculation steps of (1) measuring a diameter of a left ventricular outflow tract in an ultrasound image of a frame representing a so-called parasternal left ventricular long-axis cross section of a systolic mid-phase of the heart, and calculating a cross-sectional area of the left ventricular outflow tract, (2) calculating a velocity-time integral value of blood flow in the left ventricular outflow tract for a so-called apical five-chamber cross section or a so-called apical three-chamber cross section by a so-called pulse Doppler method, (3) calculating a so-called stroke volume as a product of the cross-sectional area of the left ventricular outflow tract and the velocity-time integral value of the blood flow in the left ventricular outflow tract, and (4) calculating the cardiac output as a product of the stroke volume and a heart rate.

In a case in which the cardiac output is calculated in this way, for example, a problem may occur in which a criterion for selecting the ultrasound image of the frame representing the systolic mid-phase of the heart varies for each user of the ultrasound diagnostic apparatus, such as a doctor, or a large amount of time is required to select the ultrasound image of the frame representing the systolic mid-phase of the heart. It is generally known that the systolic mid-phase of the heart tends to be located in the vicinity of a phase in which the diameter of the left ventricular outflow tract is maximal. JP2023-054549A discloses a technique of automatically selecting the ultrasound image of the frame in which the diameter of the left ventricular outflow tract is maximal, and for example, by using this technique, a frame in the vicinity of the phase in which the diameter of the left ventricular outflow tract is maximal can be selected.

### SUMMARY OF THE INVENTION

Here, in guidelines issued by the American Society of Echocardiography (ASE) and the like, a phase in which a so-called aortic valve annulus is most open is defined as the systolic mid-phase of the heart. Since the phase in which the aortic valve annulus is most open does not necessarily coincide with the phase in which the diameter of the left ventricular outflow tract is maximal, in a case in which the technique disclosed in JP2023-054549A is used, the ultrasound image of the frame representing the systolic mid-phase is not necessarily selected as the frame suitable for measuring the diameter of the left ventricular outflow tract, and the ultrasound image of the frame corresponding to a different phase may be selected each time the ultrasound image is selected.

The present invention has been made to solve such problems in the related art, and an object of the present invention is to provide an ultrasound diagnostic apparatus and a control method of the ultrasound diagnostic apparatus that can easily and accurately select an ultrasound image of a frame suitable for measuring a diameter of a left ventricular outflow tract.

The above object can be achieved with the following configurations.
[1] An ultrasound diagnostic apparatus that guides a frame suitable for measuring a diameter of a left ventricular outflow tract from ultrasound images of a plurality of frames in which a heart of a subject is imaged, the ultrasound diagnostic apparatus including: an image recognition unit that performs image recognition on a local anatomical structure of the heart in each of the plurality of frames; a time-varying waveform generation unit that generates a time-varying waveform of an anatomical structure state based on the local anatomical structure recognized by the image recognition unit; a candidate frame extraction unit that extracts a measurement candidate frame from the plurality of frames using the time-varying waveform generated by the time-varying waveform generation unit; a monitor; and a display controller that displays the measurement candidate frame extracted by the candidate frame extraction unit in a highlighted manner on the monitor.
[2] The ultrasound diagnostic apparatus according to [1], further including: a memory that stores a plurality of the measurement candidate frames extracted by the candidate frame extraction unit in a plurality of past cardiac cycles and the local anatomical structure recognized by the image recognition unit and corresponding to each of the measurement candidate frames.
[3] The ultrasound diagnostic apparatus according to [1] or [2], in which the image recognition unit performs image recognition on an aortic valve annulus as the local anatomical structure, the time-varying waveform generation unit generates a time-varying waveform of an angle difference with respect to a vascular running axis of the aortic valve annulus, and the candidate frame extraction unit extracts a frame in which the angle difference is minimized in the time-varying waveform as the measurement candidate frame.
[4] The ultrasound diagnostic apparatus according to [1] or [2], in which the image recognition unit performs image recognition on a mitral valve as the local anatomical structure, the time-varying waveform generation unit generates a time-varying waveform of a valve distance of the mitral valve, and the candidate frame extraction unit extracts a frame in which the valve distance is minimized in the time-varying waveform as the measurement candidate frame.
[5] The ultrasound diagnostic apparatus according to [1] or [2], in which the image recognition unit performs image recognition on a left ventricle as the local anatomical structure, the time-varying waveform generation unit generates a time-varying waveform of an area of the left ventricle, and the candidate frame extraction unit extracts the measurement candidate frame based on the time-varying waveform of the area.
[6] The ultrasound diagnostic apparatus according to [1] or [2], in which the image recognition unit performs image recognition on a left ventricle as the local anatomical structure, the time-varying waveform generation unit generates a time-varying waveform of a curvature of a contour of the left ventricle, and the candidate frame extraction unit extracts the measurement candidate frame based on the time-varying waveform of the curvature.
[7] The ultrasound diagnostic apparatus according to any one of [1] to [6], in which the display controller displays the measurement candidate frame in a highlighted manner by changing a brightness or a color of a frame line of the measurement candidate frame extracted by the candidate frame extraction unit.
[8] The ultrasound diagnostic apparatus according to any one of [1] to [6], in which the display controller displays the measurement candidate frame in a highlighted manner by changing a brightness or a color of the local anatomical structure in the measurement candidate frame extracted by the candidate frame extraction unit.
[9] The ultrasound diagnostic apparatus according to any one of [1] to [8], in which the time-varying waveform generation unit generates a plurality of time-varying waveforms related to a plurality of indices as the anatomical structure state, and the candidate frame extraction unit extracts the measurement candidate frame based on the plurality of time-varying waveforms generated by the time-varying waveform generation unit.
[10] The ultrasound diagnostic apparatus according to [2], in which the display controller displays a list of the plurality of the measurement candidate frames stored in the memory, on the monitor.
[11] The ultrasound diagnostic apparatus according to [2], further including: a frame recommendation unit that selects a frame suitable for measurement from among the plurality of measurement candidate frames by comparing the plurality of measurement candidate frames stored in the memory, and recommends the selected frame to a user.
[12] A control method of an ultrasound diagnostic apparatus that guides a frame suitable for measuring a diameter of a left ventricular outflow tract from ultrasound images of a plurality of frames in which a heart of a subject is imaged, the control method including: performing image recognition on a local anatomical structure of the heart in each of the plurality of frames; generating a time-varying waveform of an anatomical structure state based on the local anatomical structure recognized by the image recognition; extracting a measurement candidate frame from the plurality of frames using the generated time-varying waveform; and displaying the extracted measurement candidate frame in a highlighted manner on a monitor.

According to the present invention, the ultrasound diagnostic apparatus includes the image recognition unit that performs image recognition on the local anatomical structure of the heart in each of the plurality of frames, the time-varying waveform generation unit that generates the time-varying waveform of the anatomical structure state based on the local anatomical structure recognized by the image recognition unit, the candidate frame extraction unit that extracts, from the plurality of frames, the measurement candidate frame representing the systolic mid-phase by using the time-varying waveform generated by the time-varying waveform generation unit, the monitor, and the display controller that displays the measurement candidate frame extracted by the candidate frame extraction unit in the highlighted manner on the monitor. With this configuration, the ultrasound image of the frame suitable for measuring the diameter of the left ventricular outflow tract can be easily and accurately selected.

### BRIEF DESCRIPTION OF THE DRAWINGS

FIG. 1 is a block diagram showing a configuration of an ultrasound diagnostic apparatus according to Embodiment 1 of the present invention.
FIG. 2 is a block diagram showing an internal configuration of a transmission and reception circuit in Embodiment 1 of the present invention.
FIG. 3 is a block diagram showing an internal configuration of an image generation unit in Embodiment 1 of the present invention.
FIG. 4 is a diagram showing an example of an ultrasound image representing a parasternal left ventricular long-axis cross section.
FIG. 5 is a diagram showing an example of an ultrasound image of a measurement candidate frame in a highlighted manner.
FIG. 6 is a flowchart showing an operation of the ultrasound diagnostic apparatus according to Embodiment 1 of the present invention.
FIG. 7 is a diagram showing an example of ultrasound images of a plurality of measurement candidate frames displayed in a list.
FIG. 8 is a diagram showing an example of a jump button for sequentially displaying the measurement candidate frame on the monitor by skipping frames other than the measurement candidate frame.
FIG. 9 is a block diagram showing a configuration of an ultrasound diagnostic apparatus according to Embodiment 2 of the present invention.

### DESCRIPTION OF THE PREFERRED EMBODIMENTS

Hereinafter, embodiments of the present invention will be described based on the accompanying drawings.

The following configuration requirements are described based on a representative embodiment of the present invention, but the present invention is not limited to the embodiment.

In the present specification, a numerical range represented by "to" means a range including numerical values described before and after "to", both ends inclusive, as a lower limit value and an upper limit value.

In the present specification, "the same" includes an error range generally allowed in the technical field.

### Embodiment 1

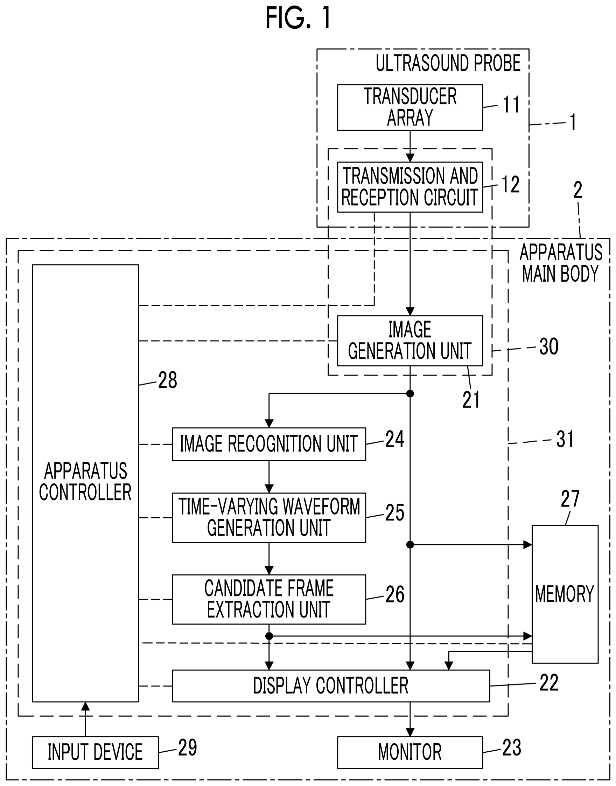

FIG. 1 shows a configuration of an ultrasound diagnostic apparatus according to Embodiment 1 of the present invention. The ultrasound diagnostic apparatus comprises an ultrasound probe 1 and an apparatus body 2 that are connected to each other by so-called wired communication or so-called wireless communication.

The ultrasound probe 1 comprises a transducer array 11 and a transmission and reception circuit 12 connected to the transducer array 11.

The apparatus main body 2 comprises an image generation unit 21 connected to the transmission and reception circuit 12. In the apparatus main body 2, a display controller 22 and a monitor 23 are sequentially connected to the image generation unit 21. In addition, an image recognition unit 24, a time-varying waveform generation unit 25, and a candidate frame extraction unit 26 are sequentially connected to the image generation unit 21. The candidate frame extraction unit 26 is connected to the display controller 22. In addition, a memory 27 is connected to the image generation unit 21 and the candidate frame extraction unit 26. The memory 27 is connected to the display controller 22. In addition, the apparatus controller 28 is connected to the transmission and reception circuit 12, the image generation unit 21, the display controller 22, the image recognition unit 24, the time-varying waveform generation unit 25, the candidate frame extraction unit 26, and the memory 27. An input device 29 is connected to the apparatus controller 28.

The transmission and reception circuit 12 and the image generation unit 21 constitute an image acquisition unit 30. In addition, the image generation unit 21, the display controller 22, the image recognition unit 24, the time-varying waveform generation unit 25, the candidate frame extraction unit 26, and the apparatus controller 28 constitute a processor 31 for the apparatus main body 2.

The transducer array 11 of the ultrasound probe 1 includes a plurality of ultrasound transducers that are one-dimensionally or two-dimensionally arranged. In accordance with a drive signal supplied from the transmission and reception circuit 12, each of the ultrasound transducers transmits ultrasound and receives an ultrasound echo from a subject to output a signal based on the ultrasound echo. Each ultrasound transducer is configured by, for example, forming electrodes at both ends of a piezoelectric material consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), and the like.

The image acquisition unit 30, which is composed of the transmission and reception circuit 12 and the image generation unit 21, acquires ultrasound images of a plurality of frames as a moving image in which a heart of the subject is imaged, by transmitting and receiving ultrasound beams using the ultrasound probe 1.

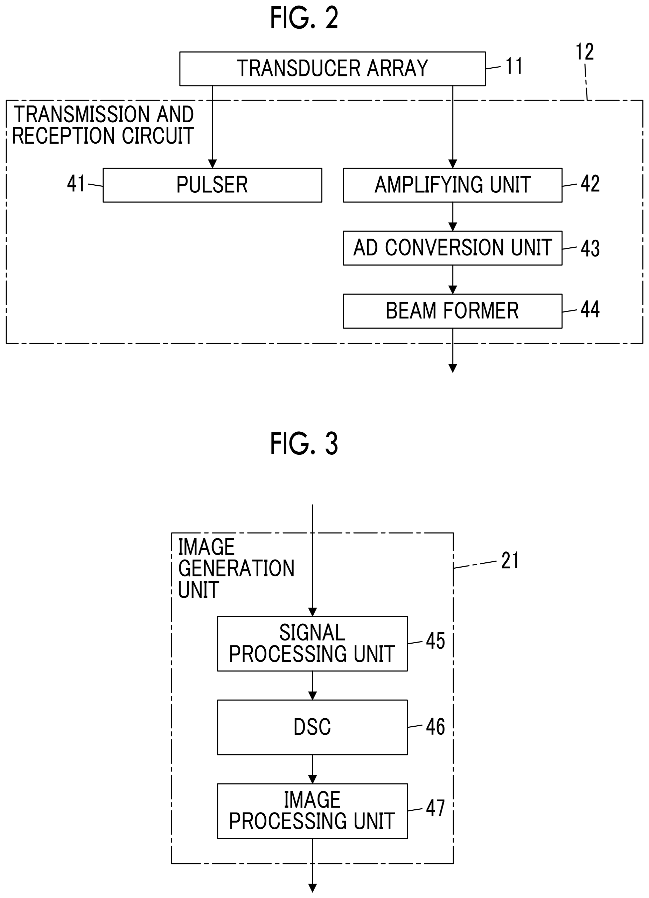

The transmission and reception circuit 12 transmits the ultrasound waves from the transducer array 11 and generates a sound ray signal based on reception signals acquired by the transducer array 11 under control of the apparatus controller 28. As shown in FIG. 2, the transmission and reception circuit 12 includes a pulser 41 connected to the transducer array 11, and an amplifying unit 42, an analog-to-digital (AD) conversion unit 43, and a beam former 44 that are sequentially connected in series to the transducer array 11.

The pulser 41 includes, for example, a plurality of pulse generators, adjusts a delay amount of each drive signal based on a transmission delay pattern selected in accordance with a control signal from the apparatus controller 28 so that the ultrasound waves transmitted from the plurality of ultrasound oscillators of the transducer array 11 form an ultrasound beam, and supplies each drive signal to the plurality of ultrasound oscillators. As described above, in a case in which a pulsed or continuous wave-like voltage is applied to the electrodes of the ultrasound transducer of the transducer array 11, the piezoelectric material expands and contracts to generate pulsed or continuous wave-like ultrasound from each of the ultrasound transducers, whereby the ultrasound beam is formed from the combined wave of the ultrasound.

The transmitted ultrasound beam is, for example, reflected by a target such as a part of the subject and propagates toward the transducer array 11 of the ultrasound probe 1. The ultrasound echo propagating toward the transducer array 11 in this way is received by each of the ultrasound transducers constituting the transducer array 11. In such a case, each of the ultrasound transducers constituting the transducer array 11 receives the propagating ultrasound echo to expand and contract, generates the reception signal, which is an electrical signal, and outputs these reception signals to the amplifying unit 42.

The amplifying unit 42 amplifies the signal input from each of the ultrasound transducers constituting the transducer array 11 and transmits the amplified signal to the AD conversion unit 43. The AD conversion unit 43 converts the signal transmitted from the amplifying unit 42 into digital reception data. The beam former 44 performs so-called reception focus processing by applying and adding the delay to each reception data received from the AD conversion unit 43. By the reception focus processing, each reception data, which is converted by the AD conversion unit 43, is phase-added, and the sound ray signal in which the focus of the ultrasound echo is narrowed down is acquired.

As shown in FIG. 3, the image generation unit 21 has a configuration in which a signal processing unit 45, a digital scan converter (DSC) 46, and an image processing unit 47 are sequentially connected in series.

The signal processing unit 45 corrects attenuation by distance of the sound ray signal received from the transmission and reception circuit 12 in accordance with depths of reflection positions of the ultrasound waves using a sound speed value set by the apparatus controller 28 and then performs envelope detection processing on the sound ray signal to generate a B-mode image signal that is tomographic image information related to tissues inside the subject.

The DSC 46 converts (raster-converts) the B-mode image signal, which is generated by the signal processing unit 45, into the image signal in accordance with a normal television signal scanning method.

The image processing unit 47 performs various types of necessary image processing such as gradation processing on the B-mode image signal input from the DSC 46, and then transmits the B-mode image signal to the display controller 22, the image recognition unit 24, and the memory 27. Hereinafter, the B-mode image signal, which is image-processed by the image processing unit 47, will be referred to as an ultrasound image.

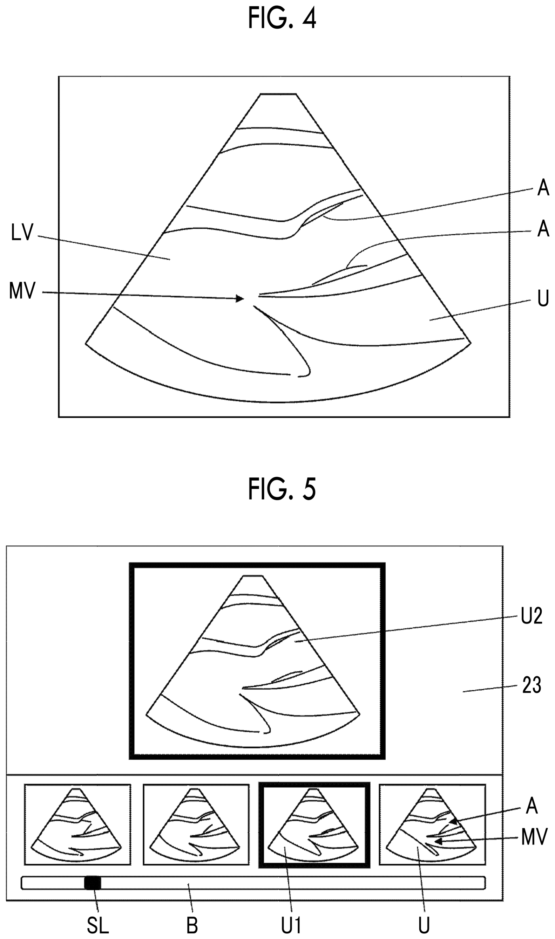

In the present invention, the image acquisition unit 30 acquires the ultrasound images of the plurality of frames representing the tomographic plane of the heart of the subject. For example, as shown in FIG. 4, an ultrasound image U representing a so-called parasternal left ventricular long-axis cross section that longitudinally passes through a so-called aortic valve annulus of the heart is acquired. The ultrasound image U representing the parasternal left ventricular long-axis cross section usually includes a left ventricular outflow tract T, an aortic valve annulus A, a part of a left ventricle LV, and a mitral valve MV.

A technique of calculating a so-called cardiac output by capturing the ultrasound image U representing the tomographic plane of the heart of the subject using the ultrasound diagnostic apparatus and analyzing the captured ultrasound image U is known. The cardiac output is usually calculated by performing calculation steps of (1) measuring a diameter of a left ventricular outflow tract T in an ultrasound image U of a frame representing a so-called parasternal left ventricular long-axis cross section of a systolic mid-phase of the heart, and calculating a cross-sectional area of the left ventricular outflow tract T, (2) calculating a velocity-time integral value of blood flow in the left ventricular outflow tract T for a so-called apical five-chamber cross section or a so-called apical three-chamber cross section by a so-called pulse Doppler method, (3) calculating a so-called stroke volume as a product of the cross-sectional area of the left ventricular outflow tract T and the velocity-time integral value of the blood flow in the left ventricular outflow tract T, and (4) calculating the cardiac output as a product of the stroke volume and a heart rate.

In a case in which the cardiac output is calculated in this way, for example, a problem may occur in which a criterion for selecting the ultrasound image U of the frame representing the systolic mid-phase of the heart varies for each user of the ultrasound diagnostic apparatus, such as a doctor, or a large amount of time is required to select the ultrasound image U of the frame representing the systolic mid-phase of the heart.

Here, in guidelines issued by the American Society of Echocardiography (ASE) and the like, in one cardiac cycle, that is, a period in which one heartbeat is performed, a phase in which a so-called aortic valve annulus A is most open is defined as a systolic mid-phase of the heart. In addition, in the systolic mid-phase of the heart, the so-called mitral valve MV is often closed, and the volume and the curvature of the left ventricle LV gradually change. Therefore, for example, the systolic mid-phase of the heart can be specified from a temporal change in a local anatomical structure state of the heart, such as an opening degree of the aortic valve annulus A.

The image recognition unit 24 performs image recognition on the local anatomical structure of the heart in each of the plurality of frames acquired by the image acquisition unit 30. Here, the local anatomical structure includes the aortic valve annulus A, the mitral valve MV, and the left ventricle LV. The image recognition unit 24 can perform image recognition of the local anatomical structure by, for example, a so-called template matching method of searching for the local anatomical structure in the ultrasound image U using template image data representing a general image or the like representing the local anatomical structure of the heart, which is stored in advance. The image recognition unit 24 can also perform image recognition of the local anatomical structure by, for example, inputting the ultrasound image U to a trained model in so-called machine learning in which the local anatomical structure of the heart is to be learned in advance.

The time-varying waveform generation unit 25 generates a time-varying waveform of the anatomical structure state based on the local anatomical structure of the heart recognized by the image recognition unit 24. The time-varying waveform generation unit 25 can generate, for example, a time-varying waveform of an angle difference with respect to a vascular running axis of the aortic valve annulus A as the time-varying waveform of the anatomical structure state. The vascular running axis refers to an axis along a running direction of an aorta in the vicinity of the aortic valve annulus A. The angle difference with respect to the vascular running axis of the aortic valve annulus A refers to an angle between the aortic valve annulus A and the vascular running axis in the ultrasound image U. In the ultrasound image U representing the parasternal left ventricular long-axis cross section, typically, a pair of aortic valve annuli A is shown on both sides of the vascular running axis. In this case, for example, an average value of the angle differences calculated for the pair of aortic valve annuli A can be calculated as the final value of the angle difference as the angle difference with respect to the vascular running axis of the aortic valve annulus A.

The time-varying waveform of the anatomical structure state refers to a waveform representing a time-series change in the anatomical structure state of the heart. The time-varying waveform generation unit 25 can generate, for example, a graph in which times at which the ultrasound images U of the plurality of frames are acquired are plotted on a horizontal axis and values of the anatomical structure state of the heart corresponding to each time are plotted on a vertical axis as the time-varying waveform of the anatomical structure state.

The time-varying waveform generation unit 25 can also generate, for example, a time-varying waveform of a valve distance of the mitral valve MV as the time-varying waveform of the anatomical structure state. The mitral valve MV is composed of two cusps of an anterior cusp and a posterior cusp from an anatomical viewpoint, and the valve distance of the mitral valve MV refers to a distance between the anterior cusp and the posterior cusp in the ultrasound image U.

The time-varying waveform generation unit 25 can also generate, for example, a time-varying waveform of an area of the left ventricle LV in the ultrasound image U as the time-varying waveform of the anatomical structure state. The time-varying waveform generation unit 25 can also generate, for example, a time-varying waveform of a curvature of a contour of the left ventricle LV in the ultrasound image U as the time-varying waveform of the anatomical structure state.

The candidate frame extraction unit 26 extracts a measurement candidate frame representing the systolic mid-phase, that is, a frame suitable for measuring the diameter of the left ventricular outflow tract T from the ultrasound image U of the plurality of frames by using the time-varying waveform of the anatomical structure state generated by the time-varying waveform generation unit 25. In the guidelines issued by the American Society of Echocardiography and the like, since the phase in which the aortic valve annulus A is most open in one cardiac cycle is defined as the systolic mid-phase of the heart, the candidate frame extraction unit 26 can extract, for example, the ultrasound image U of the frame in the phase in which the angle difference with respect to the vascular running axis of the aortic valve annulus A is minimized as the ultrasound image U of the measurement candidate frame representing the systolic mid-phase of the heart by referring to the time-varying waveform of the angle difference with respect to the vascular running axis of the aortic valve annulus A as the time-varying waveform of the anatomical structure state.

In the systolic mid-phase of the heart, the mitral valve MV is often in a state of being most closed in one cardiac cycle. Therefore, the candidate frame extraction unit 26 can also extract the ultrasound image U of the frame in which the valve distance of the mitral valve MV is minimized as the ultrasound image U of the measurement candidate frame representing the systolic mid-phase of the heart by referring to the time-varying waveform of the valve distance of the mitral valve MV as the time-varying waveform of the anatomical structure state.

In addition, in the systolic mid-phase of the heart, the volume of the left ventricle LV gradually decreases in one cardiac cycle. Therefore, the candidate frame extraction unit 26 can also extract the ultrasound image U of the measurement candidate frame representing the systolic mid-phase of the heart by referring to the time-varying waveform of the area of the left ventricle LV in the ultrasound image U as the time-varying waveform of the anatomical structure state. In this case, the candidate frame extraction unit 26 can extract, for example, the ultrasound image U of the frame of the systolic mid-phase from the trained model in machine learning that is trained using a plurality of time-varying waveforms of the area of the left ventricle LV and a plurality of ultrasound images U representing the systolic mid-phase, by inputting the time-varying waveform of the area of the left ventricle LV generated by the time-varying waveform generation unit 25 and ultrasound images U of the plurality of frames acquired by the image acquisition unit 30 to the trained model.

In addition, the candidate frame extraction unit 26 can also extract the ultrasound image U of the measurement candidate frame representing the systolic mid-phase of the heart by referring to the time-varying waveform of the curvature of the left ventricle LV in the ultrasound image U as the time-varying waveform of the anatomical structure state. In this case, the candidate frame extraction unit 26 can extract, for example, the ultrasound image U of the frame of the systolic mid-phase from the trained model in machine learning that is trained using a plurality of time-varying waveforms of the curvature of the left ventricle LV and a plurality of ultrasound images U representing the systolic mid-phase, by inputting the time-varying waveform of the curvature of the left ventricle LV generated by the time-varying waveform generation unit 25 and the ultrasound images U of the plurality of frames acquired by the image acquisition unit 30 to the trained model.

The candidate frame extraction unit 26 can extract the ultrasound image U of the measurement candidate frame representing the systolic mid-phase of the heart by using the time-varying waveform of the anatomical structure state in this way, so that the ultrasound image U of the measurement candidate frame representing the systolic mid-phase of the heart can be extracted under the same condition even in a case in which the user is different.

The memory 27 stores a plurality of ultrasound images U of the measurement candidate frames extracted by the candidate frame extraction unit 26 in a plurality of past cardiac cycles, and the local anatomical structure of the heart recognized by the image recognition unit 24 and corresponding to each of the ultrasound images U of the measurement candidate frames. For example, a recording medium, such as a flash memory, a hard disk drive (HDD), a solid state drive (SSD), a flexible disk (FD), a magneto-optical disk (MO disk), a magnetic tape (MT), a random access memory (RAM), a compact disc (CD), a digital versatile disc (DVD), a secure digital card (SD card), or a universal serial bus memory (USB memory), can be used as the memory 27.

The display controller 22 performs predetermined processing on the ultrasound image U or the like acquired by the image acquisition unit 30, and displays the processed ultrasound image U or the like on the monitor 23, under the control of the apparatus controller 28. In addition, the display controller 22 displays the measurement candidate frame extracted by the candidate frame extraction unit 26 in a highlighted manner on the monitor 23. For example, as shown in FIG. 5, the display controller 22 can display, in a highlighted manner, the ultrasound image U1 of the measurement candidate frame among the ultrasound images U of the plurality of frames in a so-called cine playback in which the ultrasound images U of the plurality of frames already acquired are sequentially played back as a video.

FIG. 5 shows an example in which an ultrasound image U2 that is sequentially displayed in time series as a video, ultrasound images U of a plurality of frames that are sequentially displayed in time series in a scroll manner, a scroll bar B having a thin and elongated shape extending along an extension direction and each position in the extension direction corresponding to each time point from a start time point of acquisition to an end time point of acquisition of the ultrasound images U of the plurality of frames, and a slider SL that moves along the extension direction on the scroll bar B in a case in which the ultrasound image U2 that is sequentially displayed in time series as a video is acquired are displayed on the monitor 23. The display controller 22 can display the ultrasound image U1 of the measurement candidate frame among the plurality of ultrasound images U of the frames that are sequentially displayed in a scroll manner in a highlighted manner. In addition, the display controller 22 can also display the ultrasound image U1 of the measurement candidate frame in a highlighted manner as the ultrasound image U2 that is sequentially displayed in time series as a video at a timing at which the ultrasound image U1 of the measurement candidate frame is displayed. In this case, the ultrasound image U2 corresponding to the ultrasound image U1 of the measurement candidate frame is also displayed in a highlighted manner. In the example of FIG. 5, ultrasound images U of four frames representing an anatomical structure of the heart that gradually changes in time series and an ultrasound image U1 of the measurement candidate frame in which the aortic valve annulus A is most open are shown.

The display controller 22 can display the ultrasound image U1 of the measurement candidate frame in a highlighted manner by making a display aspect of a frame line of the ultrasound image U1 of the measurement candidate frame different from a display aspect of a frame line of the ultrasound image U of the other frames, making a display color and a brightness of the ultrasound image U1 of the measurement candidate frame different from a display color and a brightness of the ultrasound image U of the other frames, and making a display aspect of the local anatomical structure in the ultrasound image U1 of the measurement candidate frame different from a display aspect of the local anatomical structure in the ultrasound image U of the other frames.

The display aspect of the frame line of the ultrasound image U includes a form of the frame line, such as a solid line or a dotted line, and a display color and a brightness of the frame line. In addition, the display aspect of the local anatomical structure in the ultrasound image U includes a display color and a brightness of the local anatomical structure and the presence or absence of display of a contour line of the local anatomical structure. In addition, the display controller 22 can also display the ultrasound image U1 of the measurement candidate frame in a highlighted manner by, for example, displaying a message such as "measurement candidate frame" in a so-called pop-up manner only in the vicinity of the ultrasound image U1 of the measurement candidate frame.

The user can easily and accurately select the ultrasound image U of the measurement frame representing the systolic mid-phase from the ultrasound images U of a few frames including the ultrasound image U1 of the measurement candidate frame by, for example, moving the slider SL along the extension direction of the scroll bar B via the input device 29 to check the ultrasound images U of a few frames including the ultrasound image U1 of the measurement candidate frame displayed in a highlighted manner via the input device 29.

The monitor 23 displays the ultrasound image U or the like under the control of the display controller 22 and includes, for example, a display device such as a liquid crystal display (LCD) or an organic electroluminescence display (organic EL display).

The input device 29 is for a user to perform an input operation, and is configured by, for example, a device such as a keyboard, a mouse, a trackball, a touchpad, and a touch sensor superimposed on the monitor 23.

In the present embodiment, each processing in the processor 31 is executed by any computer. In addition, any computer may execute these processes by a processor as hardware, a program as software, or a combination thereof. In such a case, the processor is configured to execute various types of processing in the present embodiment in cooperation with the program, and may function as each unit or each means in the present embodiment. In addition, the execution order of the processing by the processor is not limited to the order described above and may be changed as appropriate. Any computer may be a general-purpose computer, a computer for a specific use, a workstation, or another system capable of executing each process.

The processor 31 may be configured by one or a plurality of hardware, and the type of hardware is not limited. For example, the processor 31 may be composed of hardware such as a central processing unit (CPU), a micro processing unit (MPU), a programmable logic device such as a field programmable gate array (FPGA), a dedicated circuit for executing specific processing, such as an application specific integrated circuit (ASIC), a graphic processing unit (GPU), or a neural processing unit (NPU). In addition, the types of hardware may be a combination of different types of hardware. In a case where a plurality of hardware are configured to execute one or a plurality of processes of a certain processor, the plurality of hardware may be present in devices physically separated from each other, or may be present in the same device. In addition, in any of the embodiments, the order of each processing by the processor 31 is not limited to the above order, and may be changed as appropriate. The hardware is composed of an electric circuit (circuitry) in which circuit elements such as semiconductor elements are combined.

Furthermore, the program may be software such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be stored in a plurality of non-transitory computer-readable media existing in devices physically separated from each other. The program code or code segment may represent any combination of a procedure, a function, a subprogram, a routine, a subroutine, a module, a software package, a class, or an instruction, a data structure, or a program statement. The program code or code segment may be connected to another code segment or a hardware circuit by transmitting and receiving information, data, an argument, a parameter, or a content of a memory.

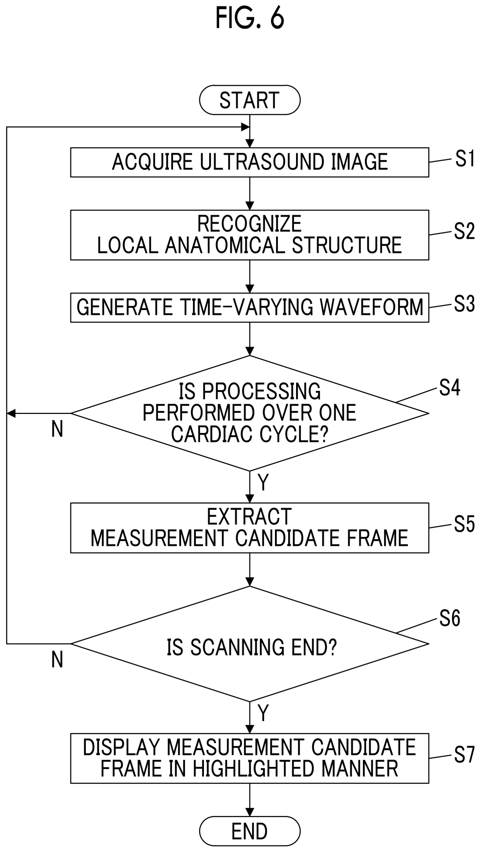

Next, an operation of the ultrasonic diagnostic apparatus according to Embodiment 1 will be described with reference to a flowchart shown in FIG. 6.

In step S1, the image acquisition unit 30 generates, for example, the ultrasound image U in which the heart of the subject representing the parasternal left ventricular long-axis cross section is imaged. In such a case, under the control of the apparatus controller 28, the transmission and reception of the ultrasound from the plurality of transducers of the transducer array 11 are started in accordance with the drive signal from the pulser 41 of the transmission and reception circuit 12 of the ultrasound probe 1, the ultrasound echo from the subject is received by the plurality of transducers of the transducer array 11, and the reception signal as the analog signal is output to the amplifying unit 42, is amplified, and then is subjected to the AD conversion via the AD conversion unit 43 to acquire the reception data.

The reception focus processing is performed on the reception data by the beam former 44, the sound ray signal generated by the reception focusing processing is transmitted to the image generation unit 21 of the apparatus main body 2, and thus the ultrasound image U representing the heart of the subject is generated by the image generation unit 21. In this case, the signal processing unit 45 of the image generation unit 21 performs the correction of the attenuation in accordance with the depth of the reflection position of the ultrasound and the envelope detection processing on the sound ray signal, the DSC 46 performs the conversion into the image signal in accordance with the normal television signal scanning method, and the image processing unit 47 performs various types of necessary image processing, such as gradation processing. The ultrasound image U generated in step S1 as described above is transmitted to the display controller 22, the image recognition unit 24, and the memory 27.

In step S2, the image recognition unit 24 recognizes the local anatomical structure of the heart, such as the aortic valve annulus A, the mitral valve MV, and the left ventricle LV, in the ultrasound image U acquired in step S1. The image recognition unit 24 can recognize the local anatomical structure of the heart by, for example, a template matching method, a method using a trained model in machine learning, or the like.

In step S3, the time-varying waveform generation unit 25 measures the anatomical structure state related to the local anatomical structure of the heart recognized by the image recognition in step S2 on the ultrasound image U, and generates the time-varying waveform of the measured anatomical structure state. The time-varying waveform generation unit 25 can measure, for example, the angle difference with respect to the vascular running axis of the aortic valve annulus A in the ultrasound image U as the anatomical structure state, and generate the time-varying waveform of the measured angle difference.

The time-varying waveform generation unit 25 can also measure, for example, the valve distance of the mitral valve MV in the ultrasound image U as the anatomical structure state, and generate the time-varying waveform of the measured valve distance. The time-varying waveform generation unit 25 can also measure, for example, the area of the left ventricle LV in the ultrasound image U as the anatomical structure state, and generate the time-varying waveform of the measured area. The time-varying waveform generation unit 25 can also measure, for example, the curvature of the contour of the left ventricle LV in the ultrasound image U as the anatomical structure state, and generate the time-varying waveform of the measured curvature.

In step S4, the candidate frame extraction unit 26 determines whether or not the processing of steps S1 to S3 is performed over one cardiac cycle of the heart of the subject by referring to the time-varying waveform generated in step S3, that is, whether or not the time-varying waveform of the anatomical structure state corresponding to one cardiac cycle is generated. For example, the candidate frame extraction unit 26 can store, in advance, template data representing a typical time-varying waveform of the anatomical structure state, and specify the time-varying waveform obtained in step S3 as one cardiac cycle of the heart in a case in which a similarity between the time-varying waveform and the template data is equal to or higher than a certain value. The processing of steps S1 to S4 is repeated as long as it is determined in step S4 that the processing is not performed over one cardiac cycle. In a case in which it is determined in step S4 that the processing is performed over one cardiac cycle, the processing proceeds to step S5.

In step S5, the candidate frame extraction unit 26 extracts the ultrasound image U1 of the measurement candidate frame representing the systolic mid-phase by using the time-varying waveform of the anatomical structure state generated in step S3. The candidate frame extraction unit 26 can extract, for example, the ultrasound image U of the frame in the phase in which the aortic valve annulus A is most open in one cardiac cycle, that is, the phase in which the angle difference with respect to the vascular running axis of the aortic valve annulus A is minimized in one cardiac cycle as the ultrasound image U1 of the measurement candidate frame. The candidate frame extraction unit 26 can extract, for example, the ultrasound image U of the frame in which the valve distance of the mitral valve MV is minimized in one cardiac cycle as the ultrasound image U1 of the measurement candidate frame representing the systolic mid-phase of the heart. In addition, the candidate frame extraction unit 26 can extract, for example, the ultrasound image U1 of the measurement candidate frame representing the systolic mid-phase of the heart based on the area of the left ventricle LV or the curvature of the left ventricle LV.

The ultrasound image U1 of the measurement candidate frame extracted in step S5 in this way is stored in the memory 27.

In step S6, the apparatus controller 28 determines whether or not to end the scanning of the heart of the subject by the ultrasound probe 1. The apparatus controller 28 can determine to end the scanning in a case in which the user inputs an instruction to end the scanning via the input device 29 by determining that a sufficient number of pieces of the ultrasound images U are acquired or the like. In addition, the apparatus controller 28 can determine to continue the scanning in a case in which the user does not input a particular instruction via the input device 29.

In a case in which it is determined to continue the scanning in step S6, the processing returns to step S1, and the processing of steps S1 to S6 is not performed again. As described above, the processing of steps S1 to S6 is repeated as long as it is determined to continue the scanning in step S6.

Here, in a case in which the processing is performed over one cardiac cycle in step S4 and the processing is performed again in step S4 by the determination in step S6 after the processing proceeds to step S5, the candidate frame extraction unit 26 sets the cardiac cycle as the determination target to the next cardiac cycle in the time series. In a case in which it is determined that the processing of steps S1 to S3 is performed over the set one cardiac cycle, the processing proceeds to step S5. In step S5, the candidate frame extraction unit 26 extracts the ultrasound image U1 of the measurement candidate frame in the set one cardiac cycle by using the time-varying waveform of the anatomical structure state in the one cardiac cycle.

In a case in which it is determined to end the scanning in step S6, the processing proceeds to step S7. In step S7, the display controller 22 displays the ultrasound image U1 of the measurement candidate frame extracted in step S5 in a highlighted manner on the monitor 23. For example, as shown in FIG. 5, the display controller 22 can display, in a highlighted manner, the ultrasound image U1 of the measurement candidate frame among the ultrasound images U of the plurality of frames in a so-called cine playback in which the ultrasound images U of the plurality of frames acquired are sequentially played back as a video.

The display controller 22 can display the ultrasound image U1 of the measurement candidate frame in a highlighted manner by, for example, making a display aspect of the frame line of the ultrasound image U1 of the measurement candidate frame and the local anatomical structure such as the aortic valve annulus A shown in the ultrasound image U1 of the measurement candidate frame different from the ultrasound image U of the other frames.

The user can check the ultrasound images U of a few frames including the ultrasound image U1 of the frame displayed in a highlighted manner in the ultrasound images U of the plurality of frames, and can easily and accurately select the ultrasound image U of the measurement frame representing the systolic mid-phase from the ultrasound images U of the frames.

In a case in which the processing of step S7 is completed in this manner, the operation of the ultrasound diagnostic apparatus according to the flowchart of FIG. 6 is completed.

As described above, according to the ultrasound diagnostic apparatus of Embodiment 1 of the present invention, since the time-varying waveform generation unit 25 generates the time-varying waveform of the anatomical structure state based on the local anatomical structure of the heart recognized by the image recognition unit 24, the candidate frame extraction unit 26 extracts the ultrasound image U1 of the measurement candidate frame representing the systolic mid-phase from the ultrasound image U of the plurality of frames by using the time-varying waveform, and the display controller 22 displays the ultrasound image U1 of the measurement candidate frame in a highlighted manner on the monitor 23, the user can easily and accurately select the ultrasound image U1 of the frame representing the systolic mid-phase of the heart.

It should be noted that a case has been described in which the transmission and reception circuit 12 is provided in the ultrasound probe 1, but the transmission and reception circuit 12 may be provided in the apparatus main body 2.

In addition, although the image generation unit 21 has been described as being provided in the apparatus main body 2, the image generation unit 21 may be provided in the ultrasound probe 1.

The apparatus main body 2 may be a so-called stationary type, a portable type that is easy to carry, or a so-called handheld type that is configured by, for example, a smartphone or a tablet type computer. As described above, the type of equipment constituting the apparatus main body 2 is not particularly limited.

Although an example has been described in which the candidate frame extraction unit 26 determines one cardiac cycle of the heart of the subject by using the template data stored in advance related to the time-varying waveform of the anatomical structure state, for example, the repetition unit of the waveform can be specified in the time-varying waveform of the period corresponding to the plurality of cardiac cycles, and the repetition unit can be specified as each one cardiac cycle.

In addition, the candidate frame extraction unit 26 can calculate a similarity between the ultrasound image U1 of the extracted one measurement candidate frame and the ultrasound image U of the frame that is sequentially generated by the image acquisition unit 30, and extract the ultrasound image U of the frame in which the calculated similarity is equal to or higher than a similarity threshold value as the ultrasound image U1 of the measurement candidate frame in each cardiac cycle.

In addition, in a case in which this processing is performed in real time while acquiring the ultrasound image U, the display controller 22 can sequentially display the ultrasound image U1 of the measurement candidate frame in a highlighted manner in real time on the monitor 23. The user can recommend the examination while checking whether or not the examination is appropriate by checking the ultrasound image U1 of the measurement candidate frame displayed in a highlighted manner for each cardiac cycle in this way.

In addition, after a sufficient number of pieces of the ultrasound images U of the frames are acquired by the image acquisition unit 30, the image recognition of the local anatomical structure of the heart in each ultrasound image U by the image recognition unit 24, the generation of the time-varying waveform of the anatomical structure state by the time-varying waveform generation unit 25, and the extraction of the ultrasound image U1 of the measurement candidate frame by the candidate frame extraction unit 26 can be performed. In this case, for example, the plurality of ultrasound images U of the frames acquired in the past examination can be stored in the memory 27 in advance, and the processing by the image recognition unit 24, the time-varying waveform generation unit 25, and the candidate frame extraction unit 26 can be performed on the plurality of ultrasound images U of the frames stored in the memory 27.

In addition, although an example has been described in which the candidate frame extraction unit 26 extracts the ultrasound image U1 of the measurement candidate frame by using the time-varying waveform of one index among the plurality of anatomical structure states such as the angle difference with respect to the vascular running axis of the aortic valve annulus A, the valve distance of the mitral valve MV, the area of the left ventricle LV, and the curvature of the left ventricle LV, for example, the measurement candidate frame can also be extracted based on a plurality of time-varying waveforms related to a plurality of indices as the anatomical structure state. The candidate frame extraction unit 26 can extract the ultrasound image U1 of the measurement candidate frame in one cardiac cycle based on each of the plurality of time-varying waveforms, and select the ultrasound image U of the frame extracted as the ultrasound image U1 of the measurement candidate frame from the largest number of time-varying waveforms in the ultrasound images U of the plurality of frames as the final ultrasound image U1 of the measurement candidate frame.

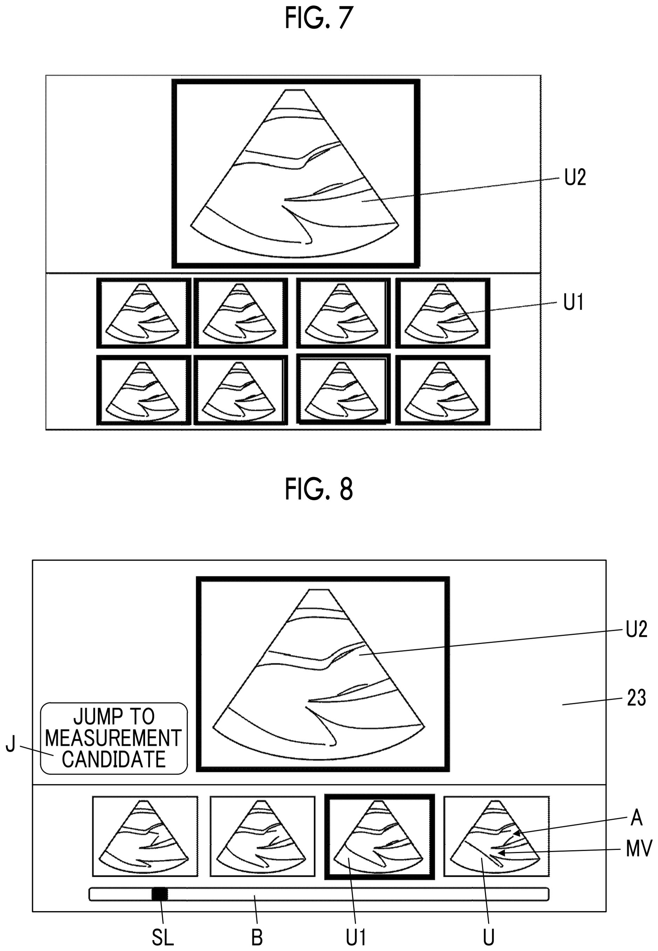

In addition, for example, as shown in FIG. 7, the display controller 22 can display a list of the ultrasound images U1 of the plurality of measurement candidate frames stored in the memory 27 on the monitor 23. The user can select the ultrasound image U of the measurement frame representing the systolic mid-phase from the ultrasound images U1 of the plurality of measurement candidate frames displayed in a list.

In addition, for example, the display controller 22 can display a jump button J as shown in FIG. 8 on the monitor 23. In a case in which the jump button J is selected by the user, the ultrasound image U1 of the measurement candidate frame and the ultrasound image U2 that is an enlarged image of the ultrasound image U1 are immediately displayed on the monitor 23, among the ultrasound images U of the plurality of frames. As a result, the user can save the time of searching for the ultrasound image U1 of the measurement candidate frame in the ultrasound images U of the plurality of frames, and can easily select the ultrasound image U of the measurement frame by checking the ultrasound images U of a few frames including the ultrasound image U1 of the measurement candidate frame.

### Embodiment 2

In a case in which the ultrasound image U1 of the measurement candidate frame is extracted in each of the plurality of cardiac cycles, the ultrasound diagnostic apparatus can also recommend the ultrasound image U of one frame to the user.

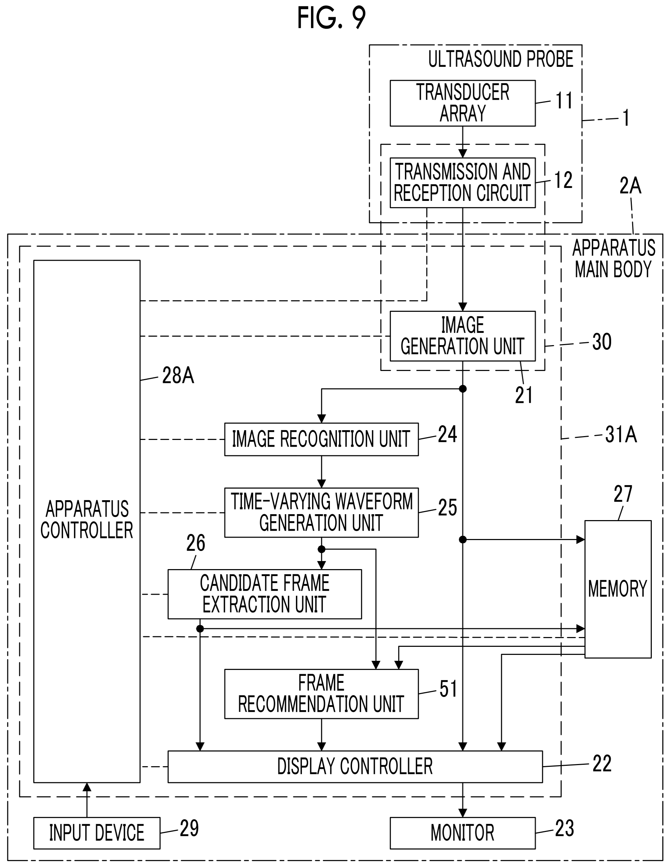

FIG. 9 shows a configuration of an ultrasound diagnostic apparatus according to Embodiment 2. The ultrasound diagnostic apparatus according to Embodiment 2 comprises an apparatus main body 2A instead of the apparatus main body 2, as compared with the ultrasound diagnostic apparatus according to Embodiment 1 shown in FIG. 1. The apparatus main body 2A further comprises a frame recommendation unit 51 and comprises an apparatus controller 28A instead of the apparatus controller 28, as compared with the apparatus main body 2 in Embodiment 1.

In the apparatus main body 2A, the frame recommendation unit 51 is connected to the time-varying waveform generation unit 25 and the memory 27. The frame recommendation unit 51 is connected to the display controller 22 and the apparatus controller 28A. In addition, the image generation unit 21, the display controller 22, the image recognition unit 24, the time-varying waveform generation unit 25, the candidate frame extraction unit 26, the apparatus controller 28A, and the frame recommendation unit 51 constitute a processor 31A for the apparatus main body 2A.

The frame recommendation unit 51 selects the ultrasound image U of the frame suitable for measurement from among the ultrasound images U1 of the plurality of measurement candidate frames by comparing the ultrasound images U1 of the plurality of measurement candidate frames stored in the memory 27, and recommends the selected ultrasound image U to the user.

In a case in which the time-varying waveform generation unit 25 generates the time-varying waveform of the angle difference with respect to the vascular running axis of the aortic valve annulus A, the frame recommendation unit 51 can select, for example, the ultrasound image U1 of the frame having the smallest angle difference among the ultrasound images U1 of the plurality of measurement candidate frames as the ultrasound image U of the frame suitable for measurement, and recommend the ultrasound image U to the user.

In addition, in a case in which the time-varying waveform generation unit 25 generates the time-varying waveform of the valve distance of the mitral valve MV, the frame recommendation unit 51 can select, for example, the ultrasound image U1 of the frame having the smallest valve distance among the ultrasound images U1 of the plurality of measurement candidate frames as the ultrasound image U of the frame suitable for measurement, and recommend the ultrasound image U to the user.

In addition, in a case in which the time-varying waveform generation unit 25 generates the time-varying waveform of the area of the left ventricle LV in the ultrasound image U, the candidate frame extraction unit 26 can calculate the ultrasound image U1 of the measurement candidate frame representing the systolic mid-phase in each cardiac cycle and a degree of certainty that is an index representing the accuracy by using the trained model in machine learning. The frame recommendation unit 51 can select, for example, the ultrasound image U1 of the frame having the largest degree of certainty among the ultrasound images U1 of the plurality of measurement candidate frames as the ultrasound image U of the frame suitable for measurement, and recommend the ultrasound image U to the user.

In addition, in a case in which the time-varying waveform generation unit 25 generates the time-varying waveform of the curvature of the left ventricle LV in the ultrasound image U, the candidate frame extraction unit 26 can calculate the ultrasound image U1 of the measurement candidate frame representing the systolic mid-phase in each cardiac cycle and a degree of certainty that is an index representing the accuracy by using the trained model in machine learning. The frame recommendation unit 51 can select, for example, the ultrasound image U1 of the frame having the largest degree of certainty among the ultrasound images U1 of the plurality of measurement candidate frames as the ultrasound image U of the frame suitable for measurement, and recommend the ultrasound image U to the user.

Since the frame recommendation unit 51 selects the ultrasound image U of the frame suitable for measurement in this way and recommends the ultrasound image U to the user, the user can easily and accurately select the ultrasound image U of the measurement frame representing the systolic mid-phase of the heart by checking the ultrasound images U of a few frames including the recommended ultrasound image U of the frame.

### Explanation of References

1: ultrasound probe
2, 2A: apparatus main body
11: transducer array
12: transmission and reception circuit
21: image generation unit
22: display controller
23: monitor
24: image recognition unit
25: time-varying waveform generation unit
26: candidate frame extraction unit
27: memory
28, 28A: apparatus controller
29: input device
30: image acquisition unit
31, 31A: processor
41: pulser
42: amplifying unit
43: AD conversion unit
44: beam former
45: signal processing unit
46: DSC
47: image processing unit
51: frame recommendation unit
A: aortic valve annulus
B: scroll bar
J: jump button
LV: left ventricle
MV: mitral valve
SL: slider
T: left ventricular outflow tract
U, U1, U2: ultrasonic image

## Claims

1. An ultrasound diagnostic apparatus that guides a frame suitable for measuring a diameter of a left ventricular outflow tract from ultrasound images of a plurality of frames in which a heart of a subject is imaged, the ultrasound diagnostic apparatus comprising:
an image recognition unit (24) that performs image recognition on a local anatomical structure of the heart in each of the plurality of frames;
a time-varying waveform generation unit (25) that generates a time-varying waveform of an anatomical structure state based on the local anatomical structure recognized by the image recognition unit (24);
a candidate frame extraction unit (26) that extracts a measurement candidate frame from the plurality of frames using the time-varying waveform generated by the time-varying waveform generation unit (25);
a monitor (23); and
a display controller (22) that displays the measurement candidate frame extracted by the candidate frame extraction unit (26) in a highlighted manner on the monitor (23).

2. The ultrasound diagnostic apparatus according to claim 1, further comprising:
a memory (27) that stores a plurality of the measurement candidate frames extracted by the candidate frame extraction unit (26) in a plurality of past cardiac cycles and the local anatomical structure recognized by the image recognition unit (24) and corresponding to each of the measurement candidate frames.

3. The ultrasound diagnostic apparatus according to claim 1 or 2,
wherein the image recognition unit (24) performs image recognition on an aortic valve annulus as the local anatomical structure,
the time-varying waveform generation unit (25) generates a time-varying waveform of an angle difference with respect to a vascular running axis of the aortic valve annulus, and
the candidate frame extraction unit (26) extracts a frame in which the angle difference is minimized in the time-varying waveform as the measurement candidate frame.

4. The ultrasound diagnostic apparatus according to claim 1 or 2,
wherein the image recognition unit (24) performs image recognition on a mitral valve as the local anatomical structure,
the time-varying waveform generation unit (25) generates a time-varying waveform of a valve distance of the mitral valve, and
the candidate frame extraction unit (26) extracts a frame in which the valve distance is minimized in the time-varying waveform as the measurement candidate frame.

5. The ultrasound diagnostic apparatus according to claim 1 or 2,
wherein the image recognition unit (24) performs image recognition on a left ventricle as the local anatomical structure,
the time-varying waveform generation unit (25) generates a time-varying waveform of an area of the left ventricle, and
the candidate frame extraction unit (26) extracts the measurement candidate frame based on the time-varying waveform of the area.

6. The ultrasound diagnostic apparatus according to claim 1 or 2,
wherein the image recognition unit (24) performs image recognition on a left ventricle as the local anatomical structure,
the time-varying waveform generation unit (25) generates a time-varying waveform of a curvature of a contour of the left ventricle, and
the candidate frame extraction unit (26) extracts the measurement candidate frame based on the time-varying waveform of the curvature.

7. The ultrasound diagnostic apparatus according to any one of claims 1 to 6,
wherein the display controller (22) displays the measurement candidate frame in a highlighted manner by changing a brightness or a color of a frame line of the measurement candidate frame extracted by the candidate frame extraction unit (26).

8. The ultrasound diagnostic apparatus according to any one of claims 1 to 6,
wherein the display controller (22) displays the measurement candidate frame in a highlighted manner by changing a brightness or a color of the local anatomical structure in the measurement candidate frame extracted by the candidate frame extraction unit (26).

9. The ultrasound diagnostic apparatus according to any one of claims 1 to 8,
wherein the time-varying waveform generation unit (25) generates a plurality of time-varying waveforms related to a plurality of indices as the anatomical structure state, and
the candidate frame extraction unit (26) extracts the measurement candidate frame based on the plurality of time-varying waveforms generated by the time-varying waveform generation unit (25).

10. The ultrasound diagnostic apparatus according to any one of claims 2 to 9,
wherein the display controller (22) displays a list of the plurality of the measurement candidate frames stored in the memory (27), on the monitor (23).

11. The ultrasound diagnostic apparatus according to any one of claims 2 to 10, further comprising:
a frame recommendation unit (51) that selects a frame suitable for measurement from among the plurality of measurement candidate frames by comparing the plurality of measurement candidate frames stored in the memory (27), and recommends the selected frame to a user.

12. A control method of an ultrasound diagnostic apparatus that guides a frame suitable for measuring a diameter of a left ventricular outflow tract from ultrasound images of a plurality of frames in which a heart of a subject is imaged, the control method comprising:
performing image recognition on a local anatomical structure of the heart in each of the plurality of frames;
generating a time-varying waveform of an anatomical structure state based on the local anatomical structure recognized by the image recognition;
extracting a measurement candidate frame from the plurality of frames using the generated time-varying waveform; and
displaying the extracted measurement candidate frame in a highlighted manner on a monitor (23).
